Europäisches Patentamt

European Patent Office

Office européen des brevets

(11) Publication number: **0 170 361**
**B1**

## EUROPEAN PATENT SPECIFICATION

(45) Date of publication of patent specification: 02.08.89

(51) Int. Cl.⁴: **A 61 K 31/55,** A 61 K 31/62

(21) Application number: 85303747.1

(22) Date of filing: 29.05.85

(54) Antiatherosclerotic agents.

(30) Priority: 06.06.84 JP 116248/84

(43) Date of publication of application:
05.02.86 Bulletin 86/06

(45) Publication of the grant of the patent:
02.08.89 Bulletin 89/31

(84) Designated Contracting States:
BE CH DE FR GB IT LI NL

(56) References cited:
GB-A-1 107 470

ARZNEIMITTEL-FORSCHUNG, vol. 22, no. 4,
April 1972, pages 639-651, Editio Cantor,
Aulendorf/Württ., DE; D. LENKE et al.: "Zur
Pharmakologie von 1,4-Bis-3-(3,4,5-
trimethoxybenzoyl-oxy)-propylr-perhydro-1,4-
diazepin (Dilazep I.N.N.), einer neuen
koronaraktiven Substanz"

(73) Proprietor: KOWA COMPANY, LTD.
6-29, 3-chome Nishiki
Naka-ku Nagoya-shi Aichi-ken (JP)

(72) Inventor: Nagakura, Masahiko
Sayamadai-Danchi 5-10-503 3-26, Sayamadai
Sayama-shi Saitama-ken (JP)
Inventor: Takimoto, Masayoshi
302-204 Kubo Hidaka-machi
Iruma-gun Saitama-ken (JP)

(74) Representative: Webb, Frederick Ronald et al
G.F. Redfern & Co. Marlborough Lodge 14
Farncombe Road
Worthing West Sussex BN11 2BT (GB)

(56) References cited:

CHEMICAL ABSTRACTS, vol. 99, no. 21, 21st
November 1983, page 44, no. 169250m,
Columbus, Ohio, US; K. NAKAJIMA et al.:
"Inhibitory effects of a combination of aspirin
and dilazep on platelet aggregation", &
YAKURI TO CHIRYO 1983, 11(6), 2107-10

**EP 0 170 361 B1**

(56) References cited:
CHEMICAL ABSTRACTS, vol. 100, no. 9, 27th February 1984, page 32, no. 61551k, Columbus, Ohio, US; S. YAMAMOTO et al.: "Platelet aggregability and aortic prostaglandin I2 production after combination treatment with dilazep and aspirin in rats and rabbits", & YAKURI TO CHIRYO 1983, 11(10), 4267-71
Pharmazeutische Stoffliste 7. Auflage Seite 70

# EP 0 170 361 B1

## Description

This invention relates to medicaments suitable for arteriosclerosis therapy and more specifically to the use of dilazep or an acid addition salt thereof, if desired together with acetylsalicylic acid, for the manufacture of such medicaments.

Arteriosclerotic diseases have in recent years accounted for one-third of all deaths in Japan. With the rapid progress of senility, a keen demand has been voiced for a method of treating arteriosclerosis.

Arteriosclerosis is a disease involving various risk factors entangled in complicated form. Hyperlipoidemia, hypertension, thrombosis and like diseases are known to be most often associated with the onset and development of arteriosclerosis and can be individually treated. However, arteriosclerosis itself cannot be treated by the existing therapeutic techniques.

Although clofibrate has been widely used as a lipid-lowering agent, the question arises whether this drug would be antiatherosclerotically active.

Research work has been done bearing in mind that an agent for use in treating arteriosclerosis must facilitate good blood supply and act directly on the metabolism in blood vessels. This invention is based on the discovery that dilazep provides significant preventive and curative benefits against experimental arteriosclerosis and that these benefits can be enhanced when dilazep is combined with acetylsalicylic acid. It has also been found that the activity mechanism of dilazep and/or acetylsalicylic acid is due to alleviated risk factors and increased protective factors, both related to arteriosclerosis, in blood vessels and in arteries.

Accordingly, one object of this invention is to provide a medicament which is effectively useful for treating arteriosclerosis as well as macroangiopathy and microangiopathy in various organs.

According to the invention, dilazep or an acid addition salt thereof is used for the manufacture of a medicament for treatment of arteriosclerosis. The dilazep may be used in combination with acetylsalicylic acid to provide an equally useful antiatherosclerotic agent.

Dilazep used as an active ingredient in this invention is a known compound that is chemically termed tetrahydro-1H-1,4-diazepine-1,4-(5H)-dipropanol bis(3,4,5-trimethoxybenzoate). Dilazep and its acid addition salts, especially in the hydrochloride form, impart increased potential to the coronary and cerebral blood flow. Despite the fact that dilazep hydrochloride has been in extensive use for the treatment of diseases such as angina pectoris and sequelae of cerebral infarction, it is surprising that this compound has direct effects on arteriosclerosis.

Acetylsalicylic acid which may be used as a coactive ingredient in the invention is a compound commonly employed in the art of medicinal products and known to strongly inhibit platelet aggregation. This compound however also inhibits the synthesis of $PGI_2$ which is believed to be one of the protective factors of arteriosclerosis.

Also surprisingly, dilazep alone or in combination with acetylsalicylic acid, is directly active against arteriosclerosis. Dilazep can arrest the inhibitory effect of acetylsalicylic acid on $PGI_2$ synthesis.

The invention will now be further described with reference to experimental examples; in these, dilazep hydrochloride and acetylsalicylic acid are referred to simply as "DZP" and "ASA", respectively.

### Experiment 1

Effect of DZP on Lipid Metabolism and Arteriosclerosis:

The effects of DZP in cholesterol-loaded rabbits were tested and evaluated under the following conditions:

Animals: New Zealand white rabbits, male, weighing about 3 kg, 8 animals in one group

Test drugs:

| | |
|---|---|
| DZP group | DZP 5 mg/kg/day |
| DZP + ASA combination group | DZP 5 mg/kg/day + ASA 0.5 mg/kg/day |
| ASA group | ASA 0.5 mg/kg/day |
| Control group | biological saline solution |

Method

Rabbits were fed with a high cholesterol diet (containing 1.5% cholesterol) for 12 consecutive weeks. The animals were subcutaneously injected with the test drugs dissolved in biological saline solution once a day as from the day of commencement of the diet. The normal group was fed with a normal diet (RC 4).

The rabbits which had fasted from the previous evening were subjected to laparotomy under pentobarbital anesthesia one hour after final administration of the drugs. Blood was collected from the inferior vena cava using 3.8% sodium citrate (containing a 1/10 volume) and the aorta was then removed. The blood and aorta thus obtained were used to conduct tests (1) to (7) below.

The numerical values were indicated as the average values plus/minus S.E.M. in tables given below.

3

The statistically significant differences from the control group were evaluated by Student's t-test at

*    P < 0.05,
**  P < 0.01 and
*** P < 0.001.

(1) Weight of Arterial Wall

Intimal media of the aorta were removed from the adventitia, followed by measurement of the weight of an upper portion of the aorta relative to the seventh bifurcation of the intercostal arteries. The results are shown in Table 1.

### Table 1

| | Weight of Aorta (mg/kg body weight) | Change (%) |
|---|---|---|
| Normal | 134 ± 8 | |
| Control | 262 ± 30 | |
| DZP | 182 ± 12* | −63 |
| DZP + ASA | 173 ± 16* | −70 |
| ASA | 231 ± 20 | −24 |

(2) Cholesterol Content in Aorta

The aorta used in test (1) was homogenized in 10 ml of a mixture of chloroform and methanol (2:1). After being allowed to stand overnight at room temperature, the homogenate was adjusted to 10 ml with the same solvent system and filtered. 50 to 200 μl of the filtrate was taken in a test tube, followed by evaporation of the solvent. Total cholesterol was determined using a Cholesterol C Test Wako (Wako Junyaku).

The results are shown in Table 2.

### Table 2

| | Cholesterol Content (mg/kg body weight) | Change (%) |
|---|---|---|
| Normal | 0.19 ± 0.02 | |
| Control | 3.37 ± 0.72 | |
| DZP | 1.71 ± 0.26* | −49 |
| DZP + ASA | 1.29 ± 0.26* | −62 |
| ASA | 2.37 ± 0.33 | −31 |

(3) Calcium Content in Aorta

The residue after the filtration procedure in test (2) was dried. To 50 mg of the dry residue was added 200 μl of 10% sodium hydroxide solution, followed by dissolution of the mixture with heating, and the

# EP 0 170 361 B1

resulting solution was partially neutralized with 45 µl of concentrated hydrochloric acid. The calcium content was determined using a calcium determination reagent, i.e. Ca Set (Iatron).

The results are shown in Table 3.

### Table 3

|  | Calcium Content (µg/mg protein) | Change (%) |
|---|---|---|
| Normal | 3.96 ± 0.34 |  |
| Control | 6.10 ± 0.66 |  |
| DZP | 4.97 ± 0.76 | -53 |
| DZP + ASA | 3.91 ± 0.32 | -102 |
| ASA | 5.81 ± 0.73 | -41 |

(4) PGI$_2$ Production in Aorta

Rings of the abdominal aorta (about 10 mg) were placed in 1 ml of Krebs-Henseleit solution and kept at 37°C for 30 minutes with stirring. A metabolite of PGI$_2$, 6-Keto-PGF$_{1\alpha}$, in the reaction mixture was determined by radioimmunoassay.

The results are shown in Table 4.

### Table 4

|  | 6-Keto-PGF$_{1\alpha}$ (ng/mg protein) | Change (%) |
|---|---|---|
| Normal | 21.6 ± 2.1 |  |
| Control | 19.5 ± 3.1 |  |
| DZP | 27.0 ± 1.5* | +38 |
| DZP + ASA | 13.3 ± 1.6 | -32 |
| ASA | 10.1 ± 1.2* | -48 |

(5) Total Cholesterol Content in Plasma

The collected plasma was adequately diluted with biological saline solution. Total cholesterol was determined for 20 µl of the plasma using a total cholesterol determination reagent (Wako Junyaku).

The results are shown in Table 5.

5

Table 5

|  | Total Cholesterol Content (mg/dl plasma) |
|---|---|
| Normal | 40 ± 6 |
| Control | 2,720 ± 260 |
| DZP | 2,370 ± 250 |
| DZP + ASA | 2,410 ± 200 |
| ASA | 2,450 ± 200 |

(6) HDL-Cholesterol Content in Plasma

HDL-cholesterol was determined for the same plasma as used in test (5) using an HDL-cholesterol determination reagent (Wako Junyaku).

The results are shown in Table 6.

Table 6

|  | HDL-Cholesterol Content (mg/dl plasma) | Change (%) |
|---|---|---|
| Normal | 19.2 ± 2.8 |  |
| Control | 22.3 ± 0.9 |  |
| DZP | 24.4 ± 0.5* | +9 |
| DZP + ASA | 27.9 ± 2.4* | +25 |
| ASA | 20.7 ± 1.8 | −7 |

(7) Thromboxane $A_2$ Production in Platelets

Sodium citrated blood was centrifuged at 200 G for 10 minutes to obtain platelet-rich plasma to which collagen (10 µg/ml) was added. A metabolite of thromboxane $A_2$, $TXB_2$ derived from 5-minute incubation, was determined by radioimmunoassay.

The results are shown in Table 7.

6

Table 7

| | TXB$_2$<br>(ng/10$^8$ platelets) | Change<br>(%) |
|---|---|---|
| Normal | 33.7 ± 3.9 | |
| Control | 28.2 ± 3.6 | |
| DZP | 31.9 ± 3.0 | +13 |
| DZP + ASA | 9.1 ± 1.6** | −67 |
| ASA | 6.0 ± 1.4*** | −79 |

As can be ascertained from tests (1) to (3), DZP inhibits the onset and development of arteriosclerosis and combination with ASA enhances its inhibitory effect. ASA alone does not function significantly as such an inhibitor.

PGI$_2$ is recognized to be important as a protective factor in arteriosclerosis. As shown in test (4), the DZP group synthesizes PGI$_2$ to a remarkably increased degree, while the ASA group substantially reduces such synthesis. However, this reduction is alleviated in the DZP—ASA combination group.

HDL-cholesterol is important as another protective factor in arteriosclerosis. As shown in test (6), both the DZP group and the DZP—ASA combination group give a marked increase in HDL-cholesterol content.

TXA$_2$ is regarded as a risk factor for platelets. As shown in test (7), the DZP—ASA combination group significantly inhibits TXA$_2$ from being synthesized.

The antiatherosclerotic activity of agents according to the invention is attributable to a mechanism different from that of clofibrate as a lipid-lowering agent. This is because test (5) shows no changes in total plasma cholesterol content in all of the DZP, ASA and DZP—ASA combination groups.

Experiment 2

Inhibitory Effect of Endothelial Permeability in Aorta and Intimal Thickening:

Rabbits, 3 animals in one group, were treated with a high cholesterol diet and with test drugs in a manner similar to that of Experiment 1. The animals were intravenously injected with 1 ml/kg of 4% Evans blue solution and subjected to laparotomy under pentobarbital anesthesia 30 minutes after injection of the drugs. Blood remaining in the inferior vena cava was washed out by perfusion of phosphate buffer solution (0.1 M, pH 7.4) containing heparin (1,000 units/l). Thereafter, the aorta was removed, immediately placed in a combination fixative for electron microscopy [neutral formalin (4%) and glutalaldehyde (1%)] and allowed to stand overnight.

Tests (1) and (2) were conducted using this aorta specimen.

(1) Effect on Endothelial Permeability of Evans Blue in Aorta

The permeability of Evans blue, i.e. the intensity of Evans blue staining, was observed on the aorta fixed in the fixative for electron microscopy. The severity was adjudged according to the grading notations of "+++", "++", "+" and "±" in the order from strong to weak staining. No staining was expressed as "−"

The results are shown in Table 8.

## Table 8

| | Animal | Evans Blue Permeability | |
|---|---|---|---|
| | No. | Aortic Arch | Thoracic Aorta |
| Normal | 1 | - | - |
| | 2 | - | - |
| | 3 | - | - |
| Control | 1 | ++ | +++ |
| | 2 | +++ | + |
| | 3 | +++ | + |
| DZP | 1 | + | ± |
| | 2 | ± | - |
| | 3 | + | - |
| DZP + ASA | 1 | + | - |
| | 2 | + | - |
| | 3 | + | - |
| ASA | 1 | + | - |
| | 2 | + | ± |
| | 3 | + | - |

(2) Effect of Intimal Thickening

Out of the aorta fixed on the fixative for electron microscopy, 8 cross-sectional tissue preparations, 2 out of the arch and 6 out of the thoracic part, were obtained and fixed with 2% osmic acid. These preparations were embedded in Epon (Shell Chemical Co.), prepared in sections or 1 μm thickness and stained with toluidine blue. The maximum thickness of each section was measured on a micrometer.

The average values of maximum thickness are shown in Table 9.

8

Table 9

| | Thickness of Intima (μm) | |
|---|---|---|
| | Aortic Arch | Thoracic Aorta |
| Normal | 18 | 7 |
| Control | 588 | 239 |
| DZP | 416 | 107 |
| DZP + ASA | 447 | 37 |
| ASA | 521 | 156 |

Elevated permeability in the aortic intima arises at an initial pathological stage of arteriosclerosis and hence is a risk factor of accelerating pathologic changes. A method of measuring the permeability of Evans blue is in common use in the art.

As shown in test (1), both the DZP group and the DZP—ASA combination group markedly inhibit the permeability of Evans blue. Test (2) shows that DZP alone and its combination with ASA considerably inhibit the intima against thickening. These results are in substantial agreement with the inhibitory effects on the weight increases in arterial walls as illustrated in test (1) of Experiment 1. This means that DZP alone or its combination with ASA is effective to inhibit elevated endothelial permeability which is a risk factor for arteriosclerosis and hence can arrest the onset and development of this disease.

It has been confirmed from the results obtained in Experiments 1 and 2 that DZP when administered alone or in combination with ASA shows a significantly excellent antiatherosclerotic activity, resulting in the inhibition of the weight increase in an arterial wall, of the thickening of intima and of the deposition of calcium and cholesterol in the arterial wall. The antiatherosclerotic agent of the invention are thus useful for the therapy of the complicated disease of arteriosclerosis since this agent is active in increasing protective factors, i.e. $PGI_2$ production in aorta and HDL-cholesterol production in plasma, and in decreasing risk factors, i.e. $TXA_2$ production in platelets and Evans blue permeability in aortic intima. Therefore, the agents of the invention can be clinically applied not only in arteriosclerosis but also in such diseases as macroangiopathy and microangiopathy in various organs including the heart, brain and kidneys.

Dilazep and acetylsalicylic acid, which are the active ingredients for the medicaments of the invention, have found extensive use in the medical field. Both of the compounds can be used over a wide range of dosages since they are believed to have no serious toxic or adverse effects. It is particularly preferred to use dilazep in the hydrochloride form.

When applied alone, dilazep is suitably dosed in a range of from 0.1 to 2,000 mg as hydrochloride per day per 1 to 4 doses per day. Acetylsalicylic acid may be combined in a range of from 0.01 to 1,000 mg with the above specified range of dilazep.

These two ingredients may be combined into a combined preparation. Where it is found desirable, the ingredients may be prepared separately and administered simultaneously. The agents of the invention may be administered either orally or parenterally. An oral route is particularly preferred.

Dilazep and acetylsalicylic acid may be applied. Simultaneously or separately in which instance these ingredients may be prepared in the form of tablets, powders, granules, capsules, injectable solutions, or suppositories. For these preparations use may suitably be made of pharmaceutically acceptable carriers or excipients such as are commonly utilized in the art.

The following examples are given to further illustrate this invention.

Example 1
Tablet Preparation of Dilazep Hydrochloride:

A plain tablet was prepared in conventional manner from a mixture of 50 mg of dilazep hydrochloride with 27 mg of corn starch, 1D mg of crystalline cellulose, 10 mg of carboxymethylcellulose, 2 mg of hydroxypropylcellulose and 1 mg of magnesium stearate. A sugar coating was then applied to the resulting tablet to produce the desired preparation.

# EP 0 170 361 B1

### Example 2
#### Granulate Preparation of Dilazep Hydrochloride:
Fifty mg of dilazep hydrochloride was mixed with 447 mg of lactose, 119 mg of corn starch, 80 mg of hardened castor oil, 40 mg of sodium carboxymethylcellulose, 119 mg of calcium carboxymethylcellulose and 45 mg of hydroxypropylcellulose. The mixture was granulated in conventional manner. A coating was then applied to the resulting granules to form the desired preparation. The preparation was 1 g per granule. The coating consisted of 52 mg of a copolymer of 2-methyl-5-vinylpyridine methacrylate and methacrylic acid, 8 mg of polyethylene glycol 6000, 2 mg of stearic acid polyoxyl 40, 8 mg of talc and 30 mg of sucrose.

### Example 3
#### Capsule Preparation of Dilazep Hydrochloride-Acetylsalicylic Acid Combination:
A mixture of 80 mg of acetylsalicylic acid with 60 mg of dilazep hydrochloride was incorporated with 30 mg of crystalline cellulose, 40 mg of corn starch and 20 mg of talc. The resulting composition was filled into a gelatinous capsule to which an enteric coating was then applied to form the desired preparation.

### Example 4
#### Tablet Preparation of Dilazep Hydrochloride-Acetylsalicylic Acid Combination
A mixture of 50 mg of acetylsalicylic acid with 37 mg of crystalline cellulose, 40 mg of calcium carboxymethylcellulose, 3 mg of hydroxypropylcellulose and 1 mg of magnesium stearate was compressed into a tablet to which an enteric coating was then applied. This tablet was used as a core tablet. Another mixture of 20 mg of dilazep hydrochloride with 154 mg of crystalline cellulose, 160 mg of lactose, 40 mg of calcium carboxymethylcellulose, 12 mg of hydroxypropylcellulose and 4 mg of magnesium stearate was compressed with the above core tablet to form the desired preparation.

### Example 5
#### Granular Preparation of Acetylsalicylic Acid:
One hundred mg of acetylsalicylic acid with 188 mg of sucrose, 100 mg of hardened castor oil and 12 mg of hydroxypropylcellulose. An enteric coating was then applied to the resulting granule to form the desired preparation.

## Claims

1. Use of dilazep or an acid addition salt thereof for the manufacture of a medicament for the treatment of arteriosclerosis.

2. Use of dilazep or an acid addition salt thereof together with acetylsalicylic acid, for the manufacture of a medicament for treatment of arteriosclerosis.

3. Use as claimed in Claim 1 or Claim 2, wherein said medicament is in the form of a tablet, powder. granule, capsule, an injectable solution or a suppository.

## Patentansprüche

1. Verwendung von Dilazep oder eines Säureadditionssalzes hiervon zur Herstellung eines Arzneimittels für die Behandlung von Arteriosklerose.

2. Verwendung von Dilazep oder eines Säureadditionssalzes hiervon, zusammen mit Acetylsalicylsäure, zur Herstellung eines Arzneimittels für die Behandlung von Arteriosklerose.

3. Verwendung nach Anspruch 1 oder 2, wobei das Arneimittel in Form von Tabletten, Pulver, Granulat, Kapseln, Injektionslösungen oder Suppositorien vorliegt.

## Revendications

1. Utilisation du dilazep ou d'un sel d'addition acide de celui-ci dans la préparation d'un médicament pour le traitement de l'artériosclérose.

2. Utilisation du dilazep ou d'un sel d'addition acide de celui-ci, en association avec l'acide acétylsalicylique dans la préparation d'un médicament pour le traitement de l'artériosclérose.

3. Utilisation selon la revendication 1 ou 2, dans laquelle ledit médicament se présente sous la forme de comprimé, de poudre, de granulé, de capsule de solution injectable ou de suppositoire.